(19) Europäisches Patentamt
European Patent Office
Office européen des brevets

(11) EP 2 219 676 B1

(12) EUROPÄISCHE PATENTSCHRIFT

(45) Veröffentlichungstag und Bekanntmachung des Hinweises auf die Patenterteilung:
**09.12.2015 Patentblatt 2015/50**

(21) Anmeldenummer: **08860544.9**

(22) Anmeldetag: **12.12.2008**

(51) Int Cl.:
***A61K 47/00*** (2006.01) ***A61L 15/44*** (2006.01)
***B01F 17/00*** (2006.01) ***C08L 33/08*** (2006.01)
***C08L 101/00*** (2006.01)

(86) Internationale Anmeldenummer:
**PCT/EP2008/010585**

(87) Internationale Veröffentlichungsnummer:
**WO 2009/074336 (18.06.2009 Gazette 2009/25)**

(54) **QUARTÄRNISIERUNG DES ZUSATZSTOFFS AMINOALKYL METHACRYLAT COPOLYMER E ZUR VERBESSERUNG DER PERMEABILITÄT UND LÖSLICHKEIT VON ARZNEISTOFFEN**

QUARTERNIZATION OF THE ADDITIVE AMINO ALKYL METHACRYLATE COPOLYMER E FOR IMPROVING PERMEABILITY AND SOLUBILITY OF PHARMACEUTICALS

QUATERNISATION DE L'ADDITIF MÉTHACRYLATE D'AMINOALKYLE COPOLYMÈRE E POUR L'AMÉLIORATION DE LA PERMÉABILITÉ ET DE LA SOLUBILITÉ DE SUBSTANCES PHARMACEUTIQUES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **13.12.2007 DE 102007060175**

(43) Veröffentlichungstag der Anmeldung:
**25.08.2010 Patentblatt 2010/34**

(73) Patentinhaber: **Evonik Röhm GmbH**
**64293 Darmstadt (DE)**

(72) Erfinder:
- **LANGGUTH, Peter**
  **63599 Biebergemünd (DE)**
- **GRUBE, Stefan**
  **10119 Berlin (DE)**
- **FREY, Holger**
  **79312 Emmendingen (DE)**
- **OBERMEIER, Boris**
  **65795 Hattersheim (DE)**

(56) Entgegenhaltungen:
**EP-A1- 0 913 158** **EP-A1- 1 810 668**
**WO-A2-02/100525** **WO-A2-2004/052099**

Bemerkungen:
Die Akte enthält technische Angaben, die nach dem Eingang der Anmeldung eingereicht wurden und die nicht in dieser Patentschrift enthalten sind.

EP 2 219 676 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung stellt eine Strategie zur Verbesserung der Permeabilität und Löslichkeit von Arzneistoffen dar, die auf dem Zusatz eines chemisch modifizierten Aminoalkyl Methacrylat Copolymer E beruht, wobei die chemische Modifizierung in der Quartämisierung eines Anteils der vorhandenen Aminoalkylgruppen besteht.

Die geringe Bioverfügbarkeit vieler Arzneistoffe stellt ein großes Problem bei der Arzneiformulierung dar. Maßgeblich für die Bioverfügbarkeit bezüglich einer bestimmten Applikationsroute sind in erster Linie die Löslichkeit und die Permeabilität des Wirkstoffs. Eine schlechte Löslichkeit bei guter Permeabilität kann ebenso zu einer schlechten Bioverfügbarkeit führen wie eine gute Löslichkeit bei schlechter Permeabilität. Zur Überwindung von Löslichkeits- und Permeabilitätsproblemen werden verschiedene Strategien verfolgt.

Die Permeabilität einer Substanz im Darm kann etwa durch den Einsatz bestimmter Hilfsstoffe erhöht werden. Solche Hilfsstoffe sind zum Beispiel Chitosan oder Natriumcaprat. Hierbei wird angenommen, dass diese Substanzen vor allem den parazellulären Transport beeinflussen (Current Drug Delivery, 2005, 2, 9-22). Denkbar ist aber auch ein positiver Einfluss auf den transzellulären Transport.

Für Aminoalkyl Methacrylat Copolymer E (Pharmacopeia japonica; als "Basic butylated methacrylate copolymer" im Europäischen Arzneibuch gelistet) konnte gezeigt werden, dass bei gleichzeitiger oraler Verabreichung mit Tetracyclin dessen AUC ("Area under the curve" = Maß für die Gesamtmenge eines Medikaments, das vom Körper aufgenommen wird) erhöht ist (EP 1302201 A1). Zudem kann Eudragit E 100, eine kommerziell erhältliche Form des Aminoalkyl Methacrylat Copolymer E (Röhm GmbH), die Komplexierung von kationischen Wirkstoffen an Mucus bzw. Gallensäuren vermindern (Takemura, Controlled Release Society 32nd Annual Meeting and Exposition; EP 1302201 A1; Macromol. Biosci., 2005, 5, 207-213). Des Weiteren konnten Alasino et al. zeigen, dass Doxorubicin-beladene Liposomen in Gegenwart von Eudragit E 100 bei unveränderter Liposomengröße mehr. Wirkstoff freisetzten als in Abwesenheit von Eudragit E 100 (Macromol. Biosci., 2005, 5, 207-213). Dies deutet auf eine permeabilitätsverändernde Wirkung von Eudragit E 100 auf Lipidmembranen hin. Der Mechanismus über den Eudragit E 100 die Bioverfügbarkeit zu steigern vermag, ist noch nicht explizit erforscht. Mögliche Mechanismen sind die Bindung von Gallensäuren, die Verhinderung der Bindung des Arzneistoffs an Mucus und die Interaktion von Eudragit E 100 mit der Zellmembran bzw. den Tight junctions.

**Abbildung 1** zeigt eine in der Literatur gängige Schreibweise von Aminoalkyl Methacrylat Copolymer E (Eudragit E). Aus ihr wird deutlich, dass es sich um ein statistisches Terpolymer und kein Triblockcopolymer handelt, d.h. die Werte von m, n und o können variieren.

Das Europäische Patent EP 1302201 A1 beschreibt den Gebrauch eines Aminoalkyl Methacrylat Copolymer E in Kombination mit einer Säure. Die Verbindung wird in Absatz [0052] näher als Eudragit E spezifiziert. Der Zusatz einer Säure wird notwendig, da diese Verbindung bei pH-Werten größer als 5,5 schlecht löslich, im ungelösten Zustand jedoch unwirksam ist.

Es war eine Aufgabe der vorliegenden Erfindung Aminoalkyl Methacrylat Copolymer E so chemisch zu modifizieren, dass seine Löslichkeit ohne den Zusatz weiterer Substanzen, wie etwa Säuren, bei pH-Werten größer als 5,5 signifikant erhöht wird. Es war eine weitere Aufgabe der vorliegenden Erfindung, dass eine solche chemische Modifikation die permeablilitätsfördernde Wirkung von Aminoalkyl Methacrylat Copolymer E nicht einschränkt. Darüber hinaus darf ein derart modifiziertes Aminoalkyl Methacrylat Copolymer E nicht toxisch sein und soll sowohl zeit- als auch kosteneffizient hergestellt werden können.

Die Aufgaben der vorliegenden Erfindung werden erfüllt durch ein Aminoalkyl Methacrylat Copolymer E, das dadurch charakterisiert ist, dass ein Anteil der Aminoalkylgruppen quartämisiert ist.

Vorzugsweise beträgt besagter Anteil der quartärnisierten Aminoalkylgruppen im Bezug auf die Gesamtzahl der Aminoalkylgruppen mehr als 10%, vorzugsweise mehr als 20%. Der Quartärnisierungsgrad ist jedoch immer weniger als 100%.

In einer bevorzugten Ausführung hat besagtes Aminoalkyl Methacrylat Copolymer E die Strukturformel:

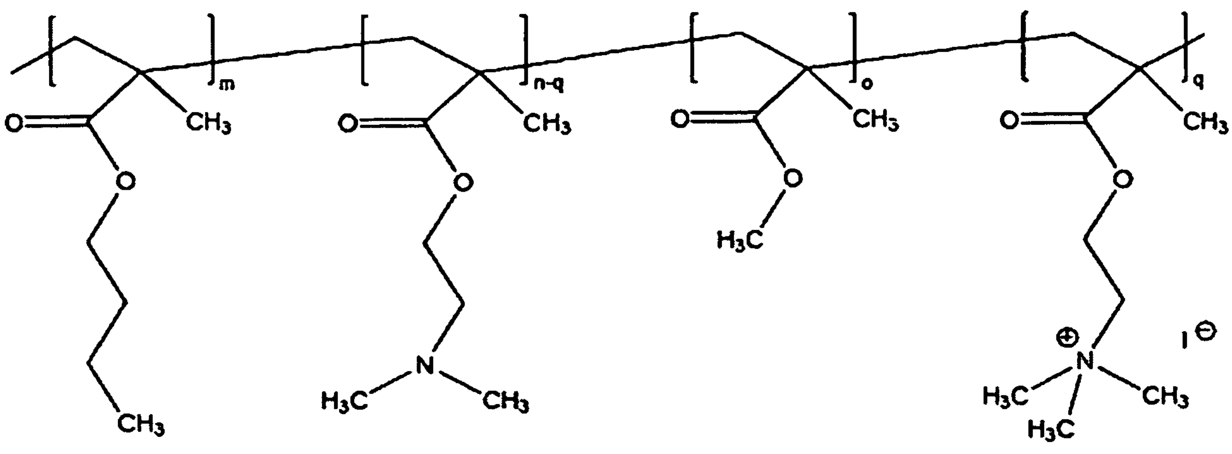

wobei m die Gesamtzahl der Butyl Methacrylatgruppen,
n die Gesamtzahl der Aminoalkylgruppen,
o die Gesamtzahl der Methyl Methacrylatgruppen, und
q die Gesamtzahl der quartärnisierten Aminoalkylgruppen bezeichnet.

Die Aufgaben der vorliegenden Erfindung werden ebenfalls erfüllt durch ein Verfahren zur Herstellung eines oben charakterisierten Aminoalkyl Methacrylat Copolymers E, wobei besagte Quartärnisierung durch Umsetzung mit einem Methylhalogenid oder Dimethylsulfat erfolgt.

In einer bevorzugten Ausführung ist besagtes Methylhalogenid ausgewählt aus der Gruppe umfassend Methyliodid, Methylbromid, Methylchlorid, vorzugsweise Methyliodid.

Vorzugsweise wird bei diesem Verfahren Methanol als Lösungsmittel verwendet.

Des Weiteren betrifft die vorliegende Erfindung die Verwendung eines oben charakterisierten Aminoalkyl Methacrylat Copolymers E zur Verbesserung der Permeabilität und Löslichkeit eines Arzneistoffs.

In einer bevorzugten Ausführung wird besagtes Copolymer zusammen mit besagtem Arzneistoff verabreicht.

Die Aufgaben der vorliegenden Erfindung werden auch durch eine pharmazeutische Formulierung erfüllt, welche einen oder mehrere Arzneistoff(e) und ein oben charakterisiertes Aminoalkyl Methacrylat Copolymer E enthält.

Die Erfinder haben überraschenderweise herausgefunden, dass quartärnisierte Derivate (Quartämisierungsgrad > 20%) des Aminoalkyl Methacrylat Copolymer E die Permeation von Substanzen mit niedriger Permeabilität transient zu steigern vermögen ohne die Barrierefunktion der Monolayer irreversibel zu beschädigen. Die Steigerungsraten sind mindestens gleich hoch, zum Teil deutlich höher als die, die mit nicht-quartämisiertem Aminoalkyl Methacrylat Copolymer E erreicht wurden.

Mit der zum Teil noch deutlich höheren permeabilitätsfördernden Wirkung des quartämisierten Aminoalkyl Methacrylat Copolymer E war von vornherein nicht zu rechnen, da von geladenen Molekülen bekannt ist, dass sie aufgrund ihrer verminderten Lipidlöslichkeit geringere Permeabilitätskoeffizienten durch Epithelien und Endothelien im Vergleich zu ihren ungeladenen Strukturanaloga aufweisen. Beispielhaft sei das Substanzpaar Scopolamin und N-Butylscopolamin genannt. Letztere Verbindung stellt eine quartäre Ammoniumverbindung (mit einer permanenten kationischen Ladung) dar, die eine nur geringe intestinale Resorptionsquote aus dem Darm und keine nennenswerte Passage durch die Blut-Hirn-Schranke aufweist. Dies ist auf die nur unzureichende Lipidlöslichkeit und Interaktion des Moleküls mit biologischen Membranen zurückzuführen.

Es konnte zudem gezeigt werden, dass der transepitheliale elektrische Widerstand (TEER) als Maß für die Durchlässigkeit der Monolayer bei Inkubation der Zellen mit quartärnisierten Polymeren auch bei pH 7,4 sinkt, während dies für das ursprüngliche Aminoalkyl Methacrylat Copolymer E nicht zutrifft. Dies lässt auf eine pH-unabhängige Löslichkeit und Wirkung der quartärnisierten Derivate schließen. Die vorliegende Erfindung erfordert damit im Gegensatz zur Lehre von EP 1302201 A1 nicht den Zusatz einer Säure. Für die orale Darreichung ergibt sich hieraus ein Vorteil, da der pH im Darm je nach Region zwischen 5,5 und 7,4 liegt. Diese Eigenschaft *könnte in vivo* eine Verbesserung der Permeation von Arzneistoffen mit geringer Permeabilität ermöglichen und damit deren Entwicklung und ihre Verwendung als Arzneimittel.

Ein Einsatz der erfindungsgemäßen Substanzen als Bioverfügbarkeitspromotoren ist für fast jede Applikationsart denkbar. Es kann sich um Lösungen, Suspensionen, Emulsionen, Inserte oder andere geeignete Arzneiformen handeln. Des Weiteren kann die Erfindung für die orale, kutane, buccale, rektale, nasale oder jede andere Applikationsart verwendet werden, bei der eine Resorptionsbarriere zu überwinden ist, um eine lokale oder systemische Wirkung eines Arzneistoffs zu ermöglichen. So ist etwa ein Einsatz für ophthalmologische Zwecke denkbar, also eine Anwendung am Auge. Hierbei könnten die Substanzen den Durchtritt bestimmter Arzneistoffe durch die Cornea verbessern.

Das erfindungsgemäße Verfahren zur Herstellung der quartärnisierten Derivate ist (auch in großem Maßstab) einfach durchzuführen und dabei zeit- und kosteneffizient.

Definitionen

Unter dem Begriff "Permeabilität" (= Durchlässigkeit), wie er hier verwendet wird, ist die Diffusion eines Stoffes, etwa eines Arzneimittels, durch Zellmembranen, insbesondere Epithelzellmembranen, zu verstehen. Die Begriffe "Zellpermeabilität" oder "epitheliale Permeabilität" können synonym verwendet werden.

Der Begriff "Quartärnisierungsgrad" bezeichnet den Anteil der quartären Stickstoffatome (oder quartären Aminoalkylgruppen) bezogen auf die Gesamtzahl der Stickstoffatome (Aminoalkylgruppen) in einer gegebenen Menge an Aminoalkyl Methacrylat Copolymer E.

Der Begriff "Arzneistoff" bezeichnet Stoffe und Zubereitungen aus Stoffen, die zur Anwendung am oder im menschlichen oder tierischen Körper bestimmt sind, um:

- Krankheiten, Leiden, Körperschäden oder krankhafte Beschwerden zu heilen, zu lindern, zu verhüten oder zu erkennen,
- Krankheitserreger, Parasiten oder körperfremde Stoffe abzuwehren, zu beseitigen oder unschädlich zu machen,
- die Beschaffenheit, den Zustand oder die Funktionen des Körpers oder seelische Zustände zu erkennen oder zu beeinflussen, und
- vom menschlichen oder tierischen Körper erzeugte Wirkstoffe oder Körperflüssigkeiten zu ersetzen.

Der Begriff "Bioverfügbarkeit" bezeichnet eine pharmakologische Messgröße für den Anteil eines Stoffes, der unverändert im systemischen Kreislauf (speziell: im Blutkreislauf) zur Verfügung steht. Sie gibt an, wie schnell und in welchem Umfang der Stoff (Arzneistoff) resorbiert wird und am Wirkungsort zur Verfügung steht.

"Eudragit E 100" (Röhm GmbH) ist eine kommerziell erhältliche Form des Aminoalkyl Methacrylat Copolymer E (alternative Bezeichnungen: Poly(Butyl Methacrylat, (2-Dimethylaminoethyl) Methacrylat, Methyl Methacrylat) und "Basic butylated methacrylate copolymer"). "Eudragit E PO" (ebenfalls Röhm GmbH) ist die Pulverform von Eudragit E 100.

Abbildungen

**Abbildung 1** zeigt die Strukturformel von Aminoalkyl Methacrylat Copolymer E (Eudragit E).

**Abbildung 2** zeigt das $^1$H NMR Spektrum (300 MHz, $MeOH_{d4}$) von Eudragit E PO (Röhm GmbH) zur Bestimmung seiner Monomerzusammensetzung. Die Signalzuordnung wird durch die Buchstaben a bis j angezeigt.

**Abbildung 3** zeigt das Reaktionsschema der Quartärnisierung von Eudragit E PO (Röhm GmbH) mit Methyliodid.

**Abbildung 4** zeigt das $^1$H NMR Spektrum (300 MHz, $MeOH_{d4}$) von Eudragit E PO (Röhm GmbH) mit einem Quartärnisierungsgrad von 0%, 22%, 42% und 65% (von unten nach oben); grau unterlegt sind die Signale der Protonen der am tertiären Stickstoff gebundenen Methyl- und Methylengruppen.

**Abbildung 5** zeigt eine Auftragung des durch $^1$H NMR-Spektroskopie bestimmten Quartärnisierungsgrades gegen die Zeit bei einer Endquartärnisierung von 65%.

**Abbildung 6** zeigt einen Graph mit den auf die Kontrolle bezogenen prozentualen Anteilen der Trypan blue-ausschließenden Caco-2-Zellen unter Angabe der aus 3 Messwerten ermittelten Standardabweichung bei pH 6,5 nach einer Inkubationszeit von 1 h.

**Abbildung 7** zeigt eine graphische Auftragung von $P_{app}$ (apperente Permeabilität) mit der Standardabweichung aus 4 Messwerten für Mannitol nach 1 h Inkubation bei pH 6,5 in Gegenwart von Eudragit E PO und seinen quartärnisierten Derivaten.

**Abbildung 8** zeigt einen Graph, der die Reversibilität der Mannitol-Permeabilität nach 1 h Inkubation mit äquimolaren Mengen an Polymer dokumentiert. $P_{app}$ wurde in Intervallen von 60 min gemessen.

**Abbildung 9** zeigt den Verlauf der TEER-Werte in Prozent des TEER-Wertes bei pH 6,5 apikal und pH 7,4 basolateral zum Zeitpunkt der Zugabe von Eudragit E PO und seinen quartärnisierten Derivaten in einer Konzentration von 0,21 μM nach einer einstündigen Inkubation in Gegenwart der Polymere und 6 h Inkubation bei pH 6,5 apikal und pH 7,4 basolateral ohne Polymerverbindungen.

**Abbildung 10** zeigt den Verlauf der TEER-Werte in Prozent des TEER-Wertes bei pH 7,4 apikal und basolateral zum Zeitpunkt der Zugabe von Eudragit E PO und seinen quartärnisierten Derivaten in einer Konzentration von 0,21 μM nach einer einstündigen Inkubation in Gegenwart der Polymere und 6 h Inkubation ohne Polymerverbindungen.

**Abbildung 11** zeigt eine graphische Auftragung von $P_{app}$ mit der Standardabweichung aus 3-5 Messwerten für Trospiumchlorid nach 2 h Inkubation bei pH 6,5 apikal und pH 7,4 basolateral und in Gegenwart von Eudragit E PO und seinen quartärnisierten Derivaten in einer Konzentration von 0,21 μM.

**Abbildung 12** zeigt eine graphische Auftragung von $P_{app}$ mit der Standardabweichung aus 3 Messwerten für Talinolol bei pH 6,5 apikal und pH 7,4 basolateral und in Gegenwart der quartärnisierten Derivaten des Eudragit E PO in einer Konzentration von 50 μg/ml.

Beispiele

1. Quartärnisierung von Eudragit E PO

Die Monomerzusammensetzung von Eudragit E PO (Röhm GmbH) wurde per $^1$H NMR-Spektroskopie bestimmt **(Abbildung 2 / Tabelle** 1). Das hierfür verwendete deuterierte Lösungsmittel $MeOH_{d4}$ wurde von Deutero GmbH bezogen.

Tabelle 1. Monomerzusammensetzung des Polymers Eudragit E PO. BMA = Butyl Methacrylat, DMAEMA = (2-Dimethylaminoethyl) Methacrylat, MMA = Methyl Methacrylat.

| | BMA (m) | DMAEMA (n) | MMA (o) |
|---|---|---|---|
| $^1$H NMR | 0,75 | 1,57 | 1 |
| Angabe des Herstellers | 1 | 2 | 1 |

Definiert quartärnisiertes Eudragit E PO wurde durch nukleophile bimolekulare Substitution an Methyliodid (Mel) durch die tertiären Amingruppen von Eudragit E PO dargestellt.

Für eine typische Quartärnisierungsreaktion **(Abbildung 3)** wurde Eudragit E PO in einem Einhalskolben in Methanol (MeOH) gelöst (0,1 g/ml; Acros Organics), in welchem sowohl die Ausgangsverbindung als auch das entsprechende quartärnisierte Produkt gut löslich sind. Die für den angestrebten Quartärnisierungsgrad benötigte Menge an Mel (Acros Organics) wurde eingewogen und zur gerührten Lösung gegeben. Bei der Methylierung wurden ca. 10% weniger Mel eingesetzt als für den angestrebten Quartärnisierungsgrad theoretisch nötig wäre, wenn man sich auf Herstellerangabe der Monomerzusammensetzung von Eudragit® EPO bezieht **(Tabelle 1).** Da der tatsächliche Stoffmengenanteil von DMAEMA gegenüber BMA und MMA zusammen 10% niedriger liegt, ist die Reduktion der Mel-Menge erforderlich. Nach 1-2 h wurde das Polymer durch langsames Zutropfen in das 20-fache Volumen stark gerührten Diethylethers bei -78 °C ausgefällt.

Zur Bestimmung des erreichten Quartärnisierungsgrades über $^1$H NMR wurde eine Probe entnommen und im Hochvakuum getrocknet. Die Quartärnisierung des Stickstoffs bzw. die Einführung einer positiven Ladung führt zu einer Verringerung der Elektronendichte an den gebunden Gruppen. Im $^1$H NMR-Spektrum zeigt sich diese Entschirmung in einer deutlichen Tieffeldverschiebung. Folglich lässt sich über die Verkleinerung der Signale der am tertiären Stickstoff gebundenen Gruppen der Quartärnisierungsgrad bestimmen. Bei Annahme stets gleicher Monomerzusammensetzung der Ausgangsverbindung wurde in allen in **Abbildung 4** gezeigten Spektren das Integral des Signals der Methylengruppe des Butylrestes des BMA bei 1,66 ppm als Referenz auf 2 gesetzt. Anschließend wurde über das Signal der Protonen der an den tertiären Stickstoff gebundenen Methylgruppen in allen Spektren exakt über denselben Abschnitt ($I_{Methyl}$) integriert,. In der Ausgangsverbindung ist der Wert dieses Integrals 9,44. Somit ergibt sich der Anteil der quartären Stickstoffatome bezogen auf die Gesamtzahl der Stickstoffatome, d.h. der Quartärnisierungsgrad $DQ_n$ nach der Formel:

$$DQ_n = 100\% - \frac{I_{Methyl} \cdot 100\%}{9{,}44}$$

$^1H$ NMR-Signale für Eudragit E PO mit einem Quartärnisierungsgrad von 65%:

$^1H$ NMR (300 MHz, $MeOH_{d4}$) $\delta$ = 4,52 (br, $COOCH_2CH_2N(CH_3)_3^+$), 4,12 (br, $COOCH_2CH_2N(CH_3)_2$), 3,91 (br, $COOCH_2CH_2CH_2CH_3$), 3,64 (br, $COOCH_3$), 3,42 (br, $COOCH_2CH_2N(CH_3)_3^+$), 2,68 (br, $COOCH_2CH_2N(CH_3)_2$), 2,35 (br, $COOCH_2CH_2N(CH_3)_2$), 2,21-1,75 (br, $CH_2$ Rückgrat), 1,65 (br, $COOCH_2CH_2CH_2CH_3$), 1,46 (br, $COOCH_2CH_2CH_2CH_3$), 1,30 (br, $CH_3$), 1,23-0,69 (br, $CH_3$, $COOCH_2CH_2CH_2CH_3$)

Zur Verfolgung des Reaktionsverlaufs wurden für eine 65%ige Quartärnisierung Proben aus dem Reaktionsgemisch entnommen. Durch Abtrennen aller flüchtigen Bestandteile aus dem Reaktionsgemisch im Hochvakuum wurde die Reaktion jeweils gestoppt. **Abbildung 5** zeigt die typische Sättigungskurve, die für die $S_N2$-Reaktion (= bimolekulare, nucleophile Substitution), die nach einer Kinetik zweiter Ordnung abläuft, zu erwarten ist. Die angestrebte Quartämisierungsgrad ist bereits nach einer Stunde erreicht, die Umsetzung verläuft quantitativ.

Nach der Quartärnisierung von Eudragit E PO mit Methyliodid ist Iodid das Gegenion zu den positiv geladenen Ammoniumgruppen. Um bei den Zellversuchen so weit wie möglich Effekte, die nicht durch die Polymere selbst verursacht werden, ausschließen zu können, wurde ein Ionenaustausch von Iodid gegen das biochemisch unbedenkliche Chlorid durchgeführt. Das Ionenaustauscherharz (Dowex Monosphere 550A, OH- beladen; Sigma Aldrich) wurde in Wasser aufgeschlämmt und in eine ca. 30 cm lange Säule mit ca. 3 cm Durchmesser gefüllt. Der pH-Wert des austretenden Wassers lag bei 8-9. Dann wurde mit halbkonzentrierter Essigsäure gespült, bis der pH-Wert im Sauren lag, und anschließend so lange mit Wasser, bis das austretende Wasser neutral war. Anschließend wurden 300 mg der quartärnisierten Verbindung in 10 ml $H_20$ auf die Säule gegeben und mit 300 ml Wasser gespült. 1 ml wurde entnommen und mit konzentrierter Silbernitratlösung (Acros Organics) versetzt. Falls ein Niederschlag zu beobachten war, wurde die Lösung wiederholt über die Säule gegeben und mit 100 ml $H_2O$ nachgespült. Der pH-Wert wurde mit HCl auf 1 eingestellt, Essigsäure und Wasser wurden abrotiert, und das Polymer bei 60 °C im Vakuumtrockenschrank mehrere Tage getrocknet. Das Endprodukt war ein farbloser Feststoff (Ausbeute: >95%).

2. Untersuchung von quartärnisierten Eudragit E PO-Derivaten im Caco-2-Zellmodell

A. Methoden

Zellkultur

Caco-2 Zellen wurden in einer Dichte von 100000 Zellen pro $cm^2$ in 24-Loch Polycarbonat-Transwells (Durchmesser: 0.33 $cm^2$) ausgesät. Die Zellen wuchsen in DMEM, welches mit 100 Einheiten/ml Penicillin, 100 mg/ml Streptomycin, 1% nichtessentielle Aminosäuren und 10% FBS angereichert war, bei 5% $CO_2$ und 90% Luftfeuchtigkeit. Die Experimente wurden 20 bis 22 Tage nach dem Aussäen ausgeführt.

Zur Aufrechterhaltung der Zellkultur wurden Zellen in 75$cm^2$ Zellkulturflaschen auf Vorrat gehalten, die mit einer Trypsin/EDTA-Lösung (0,25%/0,02%) bei 80-90% Konfluenz trypsiniert und jeden zweiten Tag mit neuem Medium versorgt wurden. Diese Zellen wurden dann wie zuvor beschrieben trypsiniert und für die Experimente in Transwells ausgesäht. Die Behandlung mit Trypsin zur Ablösung der Zellen wird auch als Passagieren bezeichnet. In diesem Sinne wurden für die Experimente lediglich Zellen der Passagen 44-58 verwendet.

Trypanblau Ausschluss-Assay

Zunächst wurde eine Lösung von 0,04% Trypanblau in 10 mM MES/HBSS (pH 6,5) hergestellt. Die Caco-2 Zellen wurden trypsiniert, zentrifugiert und in 10 mM MES/HBSS (pH 6,5) resuspendiert. Dann wurden die Zellen für 1 h in 1,5 ml Eppendorf-Gefäßen in 10 mM MES/HBSS (pH 6,5) mit 0,21 $\mu$M Polymer bei Raumtemperatur auf einem Drehrad inkubiert. 50 $\mu$l der Zellsuspension wurden anschließend mit 50 $\mu$l der Trypanblau Lösung gemischt, 20 $\mu$l dieser Mischung auf ein Hämocytometer gegeben, und die Zellen unter einem Lichtmikroskop gezählt. Intakte Zellen zeichnen sich bei diesem Test dadurch aus, dass sie den Farbstoff Trypanblau ausschließen, geschädigte/tote Zellen sind blau angefärbt.

Transport-Regenerations("transport recovery")-Assay für Mannitol

Der Assay wurde in 24-Loch Polycarbonat-Transwells durchgeführt. Die Zellen wurden für 1h mit einer Lösung des Polymers (0,21 μM), 0,1 mM Mannitol und 1 μCi/ml $^{14}C$-Mannitol in 25 mM MES/HBSS (pH 6,5) auf der apikalen Seite und 25 mM HEPES/HBSS (pH 7,4) auf der basolateralen Seite inkubiert. Die apikale und basolaterale Lösung wurden nach einer Stunde vorsichtig entfernt und durch 25 mM MES/DMEM + 10% FBS (pH 6,5) mit 0,1 mM Mannitol und 1 μCi/ml $^{14}C$-Mannitol auf der apikalen Seite und 25 mM HEPES/DMEM + 10% FBS (pH 7,4) auf der basolateralen Seite ersetzt. Die Polycarbonat-Inserts wurden stündlich in eine neue, mit Puffer gefüllte 24-Loch-Platte überführt. Proben des basolateralen Mediums wurden für die Szintillationszählung verwendet. Zur Bestimmung der initialen Mannitol-Konzentration wurden Proben des apikalen Mediums zu Beginn des Experiments und nach dem Austausch des apikalen Mediums entnommen und für die Szintillationszählung verwendet.

Transport-Assay für Trospium

Der Trospium- Transport wurde mittels HPLC analysiert. Die apikale Zellseite wurde mit einem Transportpuffer bestehend aus 10 mM MES/HBSS (pH 6,5) inkubiert, der die Polymere in einer Konzentration von 0,21 μM und 2 mM Trospium enthielt, und die basolaterale Zellseite wurde in 10 mM HEPES/HBSS (pH 7.4) inkubiert. Von der apikalen Seite wurden zu Beginn des Experiments 100 μl Proben gezogen und durch 100 μl frischen Puffer ersetzt Nach 120 min wurde der Transportversuch durch Entfernen der Filtereinheiten gestoppt und und Proben von der basolateralen Seite genommen. Bis zur Vermessung der Proben per HPLC wurden die Proben bei -18°C aufbewahrt.

Transport-Assay für Talinolol

Talinolol wurde bei einer Konzentration von 1 μCi/ml verwendet. Die apikale Seite wurde mit Polymer-Lösung (0,21μM) in 10 mM MES/HBSS-Puffer (pH 6,5) und die basolaterale Seite in 10 mM HEPES/HBSS-Puffer (pH 7,4) inkubiert. Zu Beginn wurden 20 μl Proben von der apikalen Seite entnommen und die Donor-Kammer mit 20 μl frischer Lösung wieder aufgefüllt. Nach 30, 60, 90 und 120 min wurden der basolateralen Kammer 500 μl Proben entnommen und jeweils durch 500 μl frischen Puffer ersetzt.

Szintillationszählung

Die Proben wurden in Mini Vials A-Röhrchen (Carl Roth GmbH & Co, Karlsruhe, Deutschland) mit Hilfe eines Flüssig-Szintillationszählers (LC 6000, Beckman Coulter, Unterschleißheim, Deutschland) nach gründlicher Mischung mit 4 ml Szintillationslösung Rotiszint 22 (Carl Roth GmbH & Co, Karlsruhe, Deutschland) analysiert. Die Zähldauer war für alle Proben und Experimente auf 5 min eingestellt.

HPLC

Die Messungen wurden mittels eines Jasco HPLC-Systems, bestehen aus einer Jasco PU-980 Pumpe, einem Jasco AS-950 Probensammler (Autosampler) und einem Jasco UV-975 UV/VIS Detektor (Jasco Deutschland GmbH, Groß-Umstadt, Deutschland), unter Verwendung von Ameziniummetilsulfat als internen Standard durchgeführt.
Chromatographie-Bedingungen:

*Säule:* LiChroCart 125x4 mm, RP-8, Superspher 60 (Merck Darmstadt, Deutschland)
*Mobile Phase*: 0,01 M HEPES, 0,003 M $K_2HPO_4x3H_2O$, 300 ml bidestilliertes Wasser, 700 ml Acetonitril, 1,5 ml 85% Phosphorsäure
*Temperatur:* Raumtemperatur
*Flussrate:* 1,2 ml/min
*Detektion:* UV-Absorption 210 nm
*Injektionsvolumen:* 50 μl
*Laufzeit:* 7 min

Messung des transepithelialen elektrischen Widerstandes (TEER = transepithelial electrical resistance)

Die Messung des TEER erfolgte mit einer sogenannten "Chopstick"- Elektrode (Millicell ERS, Millipore, Bedford, USA). Für die Transport-Regenerationversuche wurden 24-Loch Polykarbonat Transwells benutzt und die Zellen zunächst 20 Minuten apikal mit 25 mM MES/DMEM (pH 6.5) und basolateral mit 25 mM HEPES/DMEM + 10% FBS (pH 7.4) inkubiert. Danach wurden konzentrierte Polymerlösungen zupittetiert, so dass die Endkonzentration der Polymere

in den Inkubationsmedien 0,21 μM betrug. Der TEER wurde dann über eine Stunde verfolgt, wonach das Medium auf der apikalen Seite durch frischen Puffer (25 mM MES/DMEM (pH 6.5) + 10% FBS bzw. 25 mM HEPES/DMEM (pH 7.4) + 10% FBS) ersetzt wurde. Die Regeneration des TEER wurde dann über 6 Stunden verfolgt.

Berechnung von $P_{app}$ (apparente Permeation)

Die $P_{app}$-Werte wurden mittels folgender Formel berechnet: $P_{app} = (V_a / (A * t)) * ([Arznei]_{Akzeptor} / [Arznei]_{Start,Donor})$, wobei $V_a$ das apikale Volumen in der Akzeptorkammer in ml, A die Fläche des Monolayers in $cm^2$, t die Zeit in s, $[Arznei]_{Akzeptor}$ die kumulative Arzneikonzentration in der basolateralen Kammer nach t Sekunden und $[Arznei]_{Start,Donor}$ die Anfangsarzneikonzentration in der Donor-Kammer darstellen.

B. Ergebnisse

Von den gemäß Punkt 1 synthetisierten Verbindungen wurden drei Derivate mit einem Quartärnisierungsgrad von 22%, 42% und 65%, im Caco-2 Modell (J Pharm Sci., 2000, 89, 63-75) untersucht.

Zur Untersuchung der Toxizität der quartärnisierten Derivate von Eudragit E PO wurde zunächst ein Trypanblau Ausschluss-Test durchgeführt. Wie in **Abbildung 6** zu sehen ist, weisen die quartärnisierten Verbindungen nur eine wenig höhere Toxizität als Eudragit E PO auf. Zudem waren sowohl die Transepithelial Resistance (TEER) als auch die Erhöhung des Mannitol Transport bei Inkubation der Zellen mit den quartärnisierten Verbindungen reversibel **(Abbildungen 8** und **9).** Dies zeigt, dass das Epithel durch die Substanzen keine irreversiblen Schäden erleidet und nach Entfernen der Polymerlösungen die Barrierefunktion wiederhergestellt wird.

Bei pH 7,4 (apikal und basolateral) verhielt sich Eudragit E PO in äquimolarer Konzentration zu den quartärnisierten Derivaten bezüglich der TEER vergleichbar der Kontrolle während die quartärnisierten Verbindungen eine eindeutige Senkung der TEER verursachten **(Abbildung 10).** Möglicherweise ist Eudragit E PO bei neutralem pH wegen seiner schlechteren Löslichkeit nicht wirksam.

Neben der Erhöhung der Mannitolpermeabilität **(Abbildung 7)** erhöhten die Polymere im Zellkulturmodell auch die Permeabilität von Trospium **(Abbildung 11)** und Talinolol **(Abbildung 12).** Im Falle der Mannitol und Trospiumpermeabilität wurden äquimolare Mengen der quartärnisierten Derivate mit Eudragit E PO verglichen. Hierbei zeigte sich, dass die Derivate in äquimolarer Menge die Permeabilität der genannten Substanzen zum Teil stärker, auf jeden Fall aber mindestens genauso stark erhöhen wie Eudragit E PO.

## Patentansprüche

1. Aminoalkyl Methacrylat Copolymer E, dadurch charakterisiert, dass ein Anteil der Aminoalkylgruppen quartärnisiert ist.

2. Aminoalkyl Methacrylat Copolymer E nach Anspruch 1, wobei besagter Anteil der quartärnisierten Aminoalkylgruppen im Bezug auf die Gesamtzahl der Aminoalkylgruppen mehr als 10%, vorzugsweise mehr als 20% beträgt.

3. Verfahren zur Herstellung eines Aminoalkyl Methacrylat Copolymer E nach einem der Ansprüche 1 bis 2, wobei besagte Quartärnisierung durch Umsetzung mit einem Methylhalogenid oder Dimethylsulfat erfolgt.

4. Verfahren nach Anspruch 3, wobei besagtes Methylhalogenid ausgewählt ist aus der Gruppe umfassend Methyliodid, Methylbromid, Methylchlorid, vorzugsweise Methyliodid.

5. Verfahren nach Anspruch 3 oder 4, wobei Methanol als Lösungsmittel verwendet wird.

6. Verwendung eines Aminoalkyl Methacrylat Copolymer E nach Anspruch 1 oder 2 zur Verbesserung der Permeabilität und Löslichkeit eines Arzneistoffs.

7. Verwendung nach Anspruch 6, wobei besagtes Copolymer zusammen mit besagtem Arzneistoff verabreicht wird.

8. Pharmazeutische Formulierung, enthaltend einen oder mehrere Arzneistoff(e) und ein Aminoalkyl Methacrylat Copolymer E nach Anspruch 1 oder 2.

## Claims

1. Aminoalkyl methacrylate copolymer E, **characterized in that** a proportion of the aminoalkyl groups is quaternized.

2. Aminoalkyl methacrylate copolymer E according to Claim 1, wherein said proportion of the quaternized aminoalkyl groups relative to the total number of aminoalkyl groups is more than 10%, preferably more than 20%.

3. Method of production of an aminoalkyl methacrylate copolymer E according to either of Claims 1 to 2, wherein said quaternization takes place by reaction with a methyl halide or dimethyl sulfate.

4. Method according to Claim 3, wherein said methyl halide is selected from the group encompassing methyl iodide, methyl bromide, methyl chloride, preferably methyl iodide.

5. Method according to Claim 3 or 4, wherein methanol is used as solvent.

6. Use of an aminoalkyl methacrylate copolymer E according to Claim 1 or 2 for improving the permeability and solubility of a pharmaceutical.

7. Use according to Claim 6, wherein said copolymer is administered together with said pharmaceutical.

8. Pharmaceutical formulation comprising one or more pharmaceuticals and an aminoalkyl methacrylate copolymer E according to Claim 1 or 2.

## Revendications

1. Copolymère de méthacrylate d'aminoalkyle E, **caractérisé en ce qu'**une partie des groupes aminoalkyle est quaternisée.

2. Copolymère de méthacrylate d'aminoalkyle E selon la revendication 1, ladite proportion des groupes aminoalkyle quaternisés par rapport au nombre total de groupes aminoalkyle étant supérieure à 10%, de préférence supérieure à 20%.

3. Procédé pour la préparation d'un copolymère de méthacrylate d'aminoalkyle E selon l'une quelconque des revendications 1 à 2, ladite quaternisation ayant lieu par transformation avec un halogénure de méthyle ou un sulfate de diméthyle.

4. Procédé selon la revendication 3, ledit halogénure de méthyle étant choisi dans le groupe comprenant l'iodure de méthyle, le bromure de méthyle, le chlorure de méthyle, de préférence l'iodure de méthyle.

5. Procédé selon la revendication 3 ou 4, du méthanol étant utilisé comme solvant.

6. Utilisation d'un copolymère de méthacrylate d'aminoalkyle E selon la revendication 1 ou 2 pour améliorer la perméabilité et la solubilité d'un médicament.

7. Utilisation selon la revendication 6, ledit copolymère étant administré ensemble avec ledit médicament.

8. Formulation pharmaceutique, contenant un ou plusieurs médicaments et un copolymère de méthacrylate d'aminoalkyle E selon la revendication 1 ou 2.

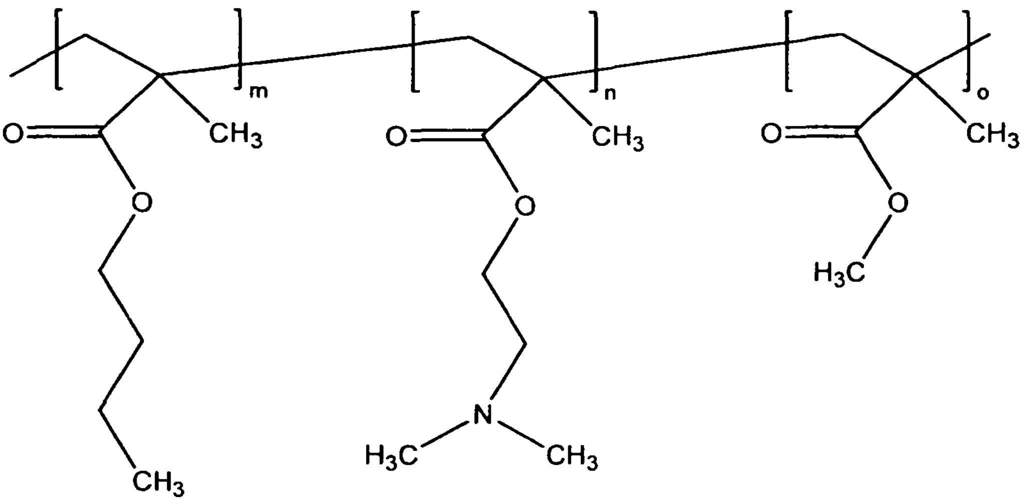

Abbildung 1

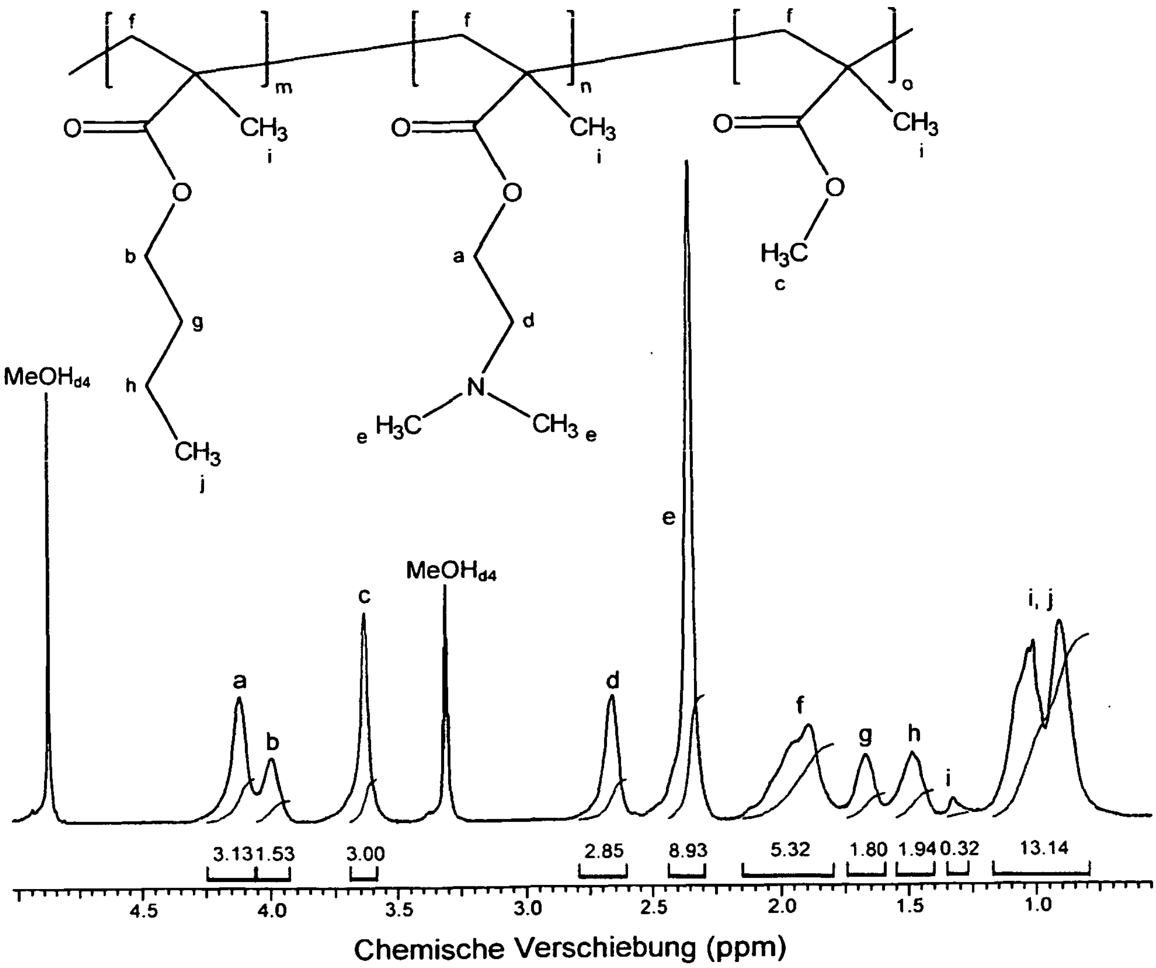
MeOHd4
e
MeOHd4
c
i, j
a
d
f
b
g
h
i
3.13 1.53
3.00
2.85
8.93
5.32
1.80
1.94
0.32
13.14
4.5
4.0
3.5
3.0
2.5
2.0
1.5
1.0
Chemische Verschiebung (ppm)

Abbildung 2

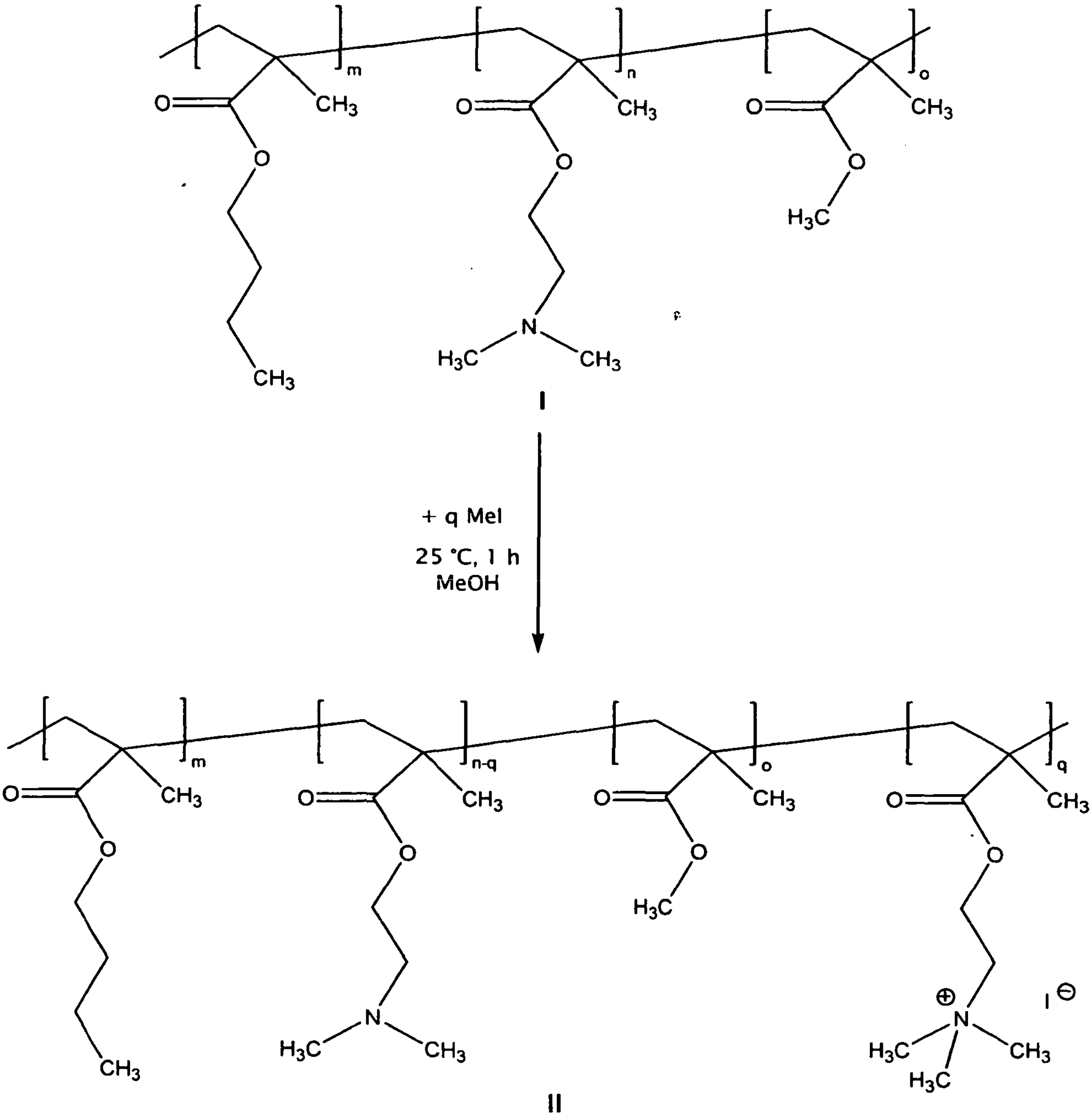


I

II

Abbildung 3

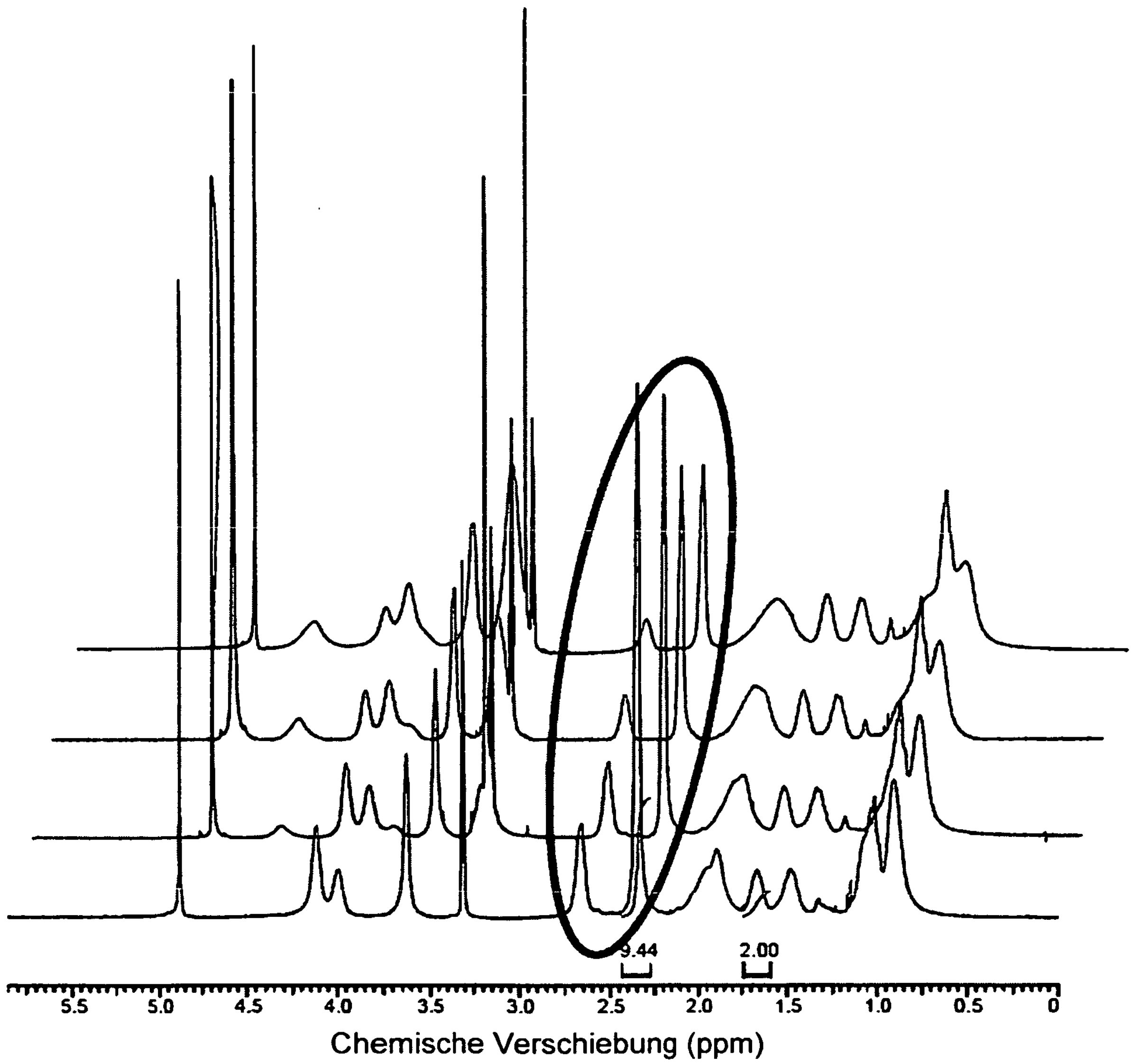
9.44
2.00
5.5
5.0
4.5
4.0
3.5
3.0
2.5
2.0
1.5
1.0
0.5
0
Chemische Verschiebung (ppm)

Abbildung 4

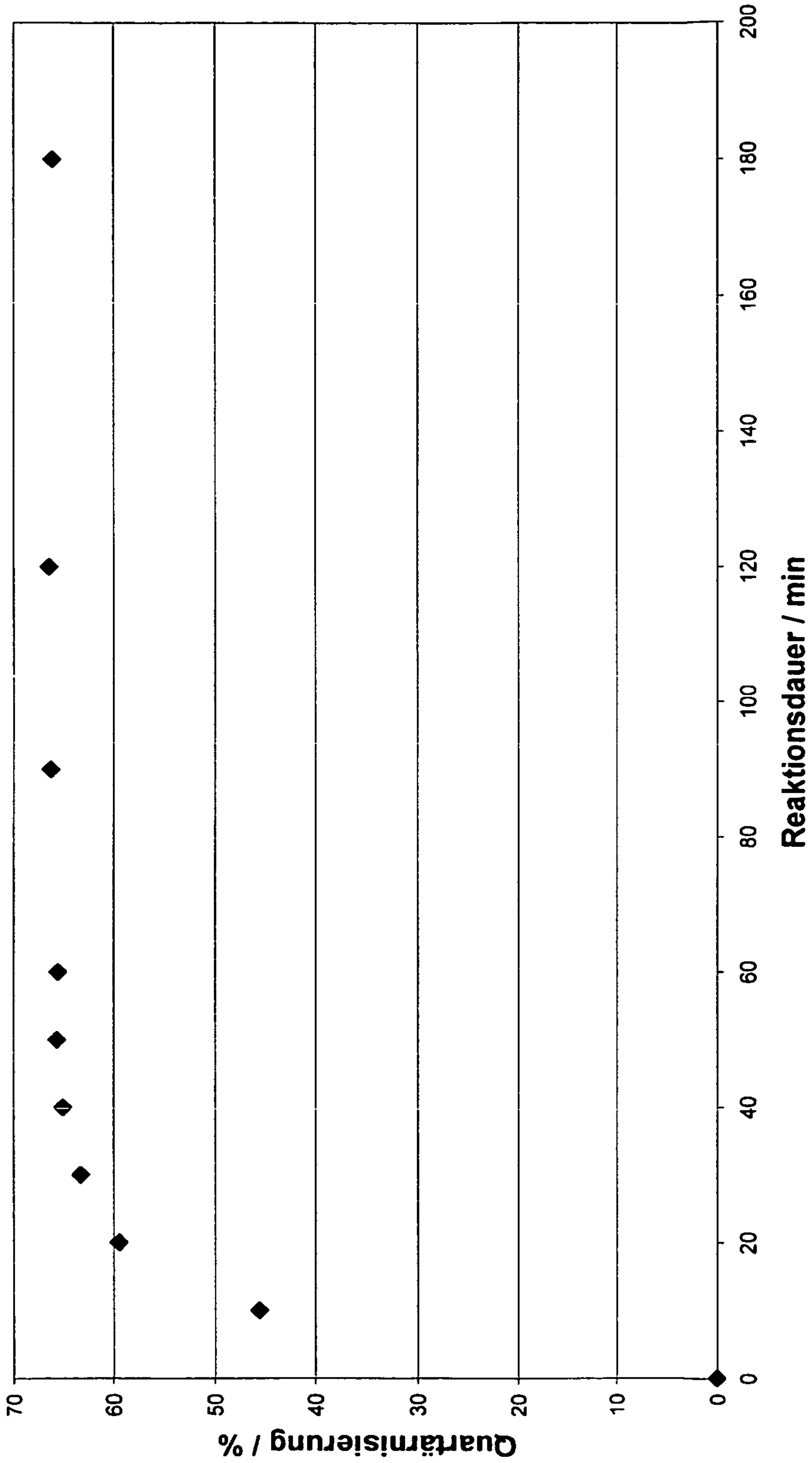

Quartärnisierung / %
0
10
20
30
40
50
60
70
Reaktionsdauer / min
0
20
40
60
80
100
120
140
160
180
200


Abbildung 5

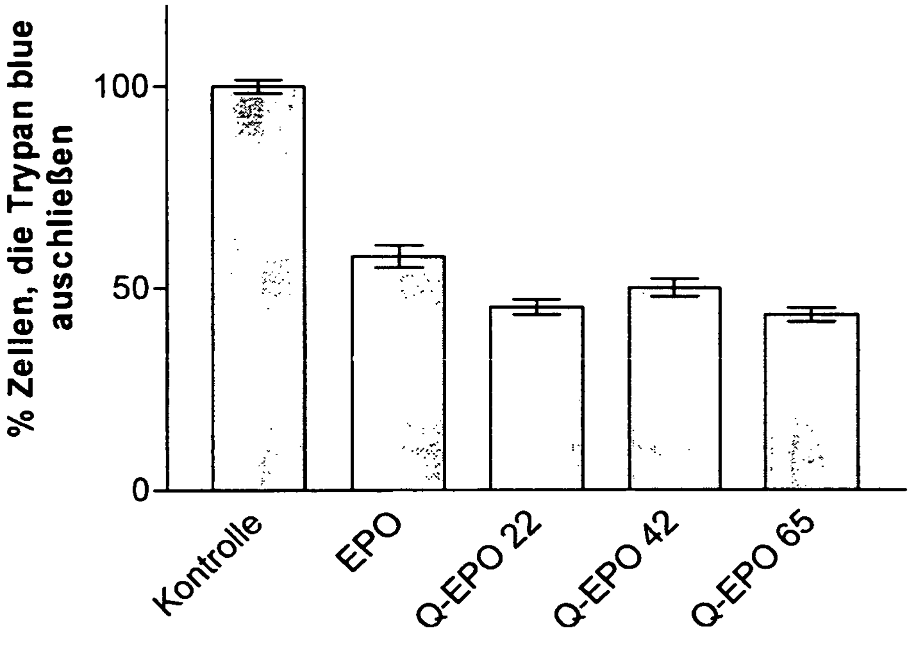
% Zellen, die Trypan blue ausschließen
100
50
0
Kontrolle
EPO
Q-EPO 22
Q-EPO 42
Q-EPO 65

Abbildung 6

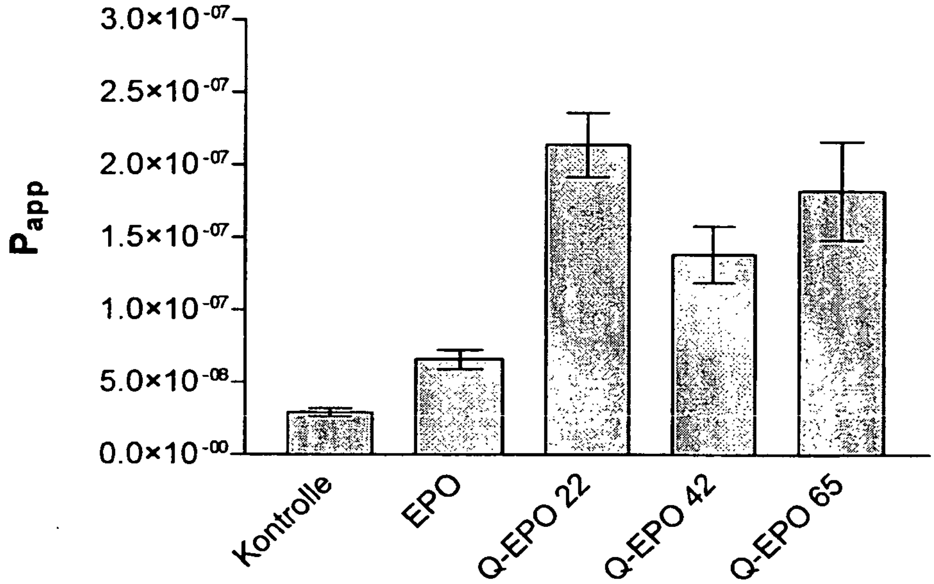
3.0×10⁻⁰⁷
2.5×10⁻⁰⁷
2.0×10⁻⁰⁷
1.5×10⁻⁰⁷
1.0×10⁻⁰⁷
5.0×10⁻⁰⁸
0.0×10⁻⁰⁰
Papp
Kontrolle
EPO
Q-EPO 22
Q-EPO 42
Q-EPO 65

Abbildung 7

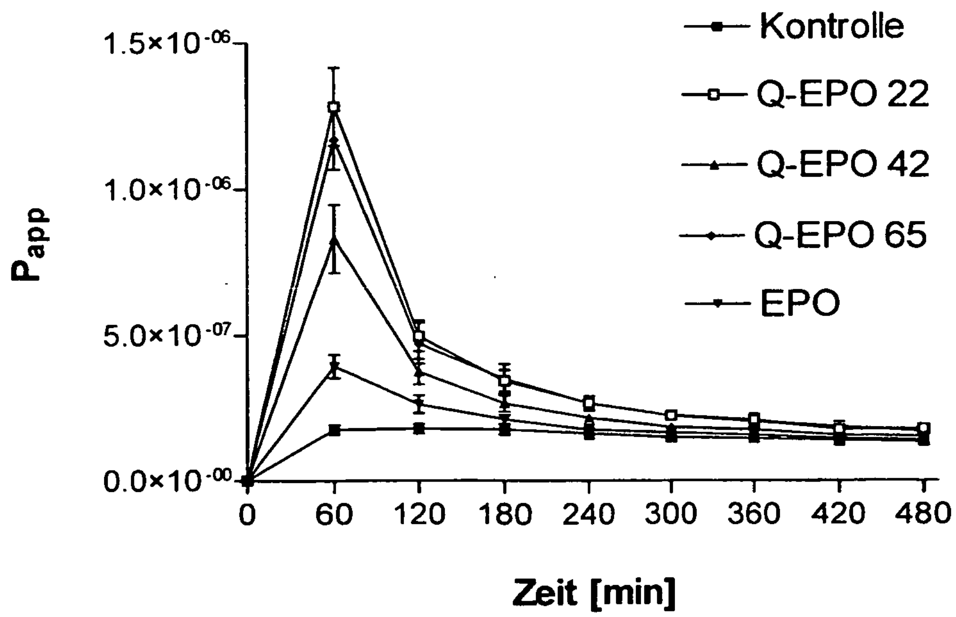

Kontrolle
Q-EPO 22
Q-EPO 42
Q-EPO 65
EPO
1.5×10-06
1.0×10-06
5.0×10-07
0.0×10-00
Papp
0
60
120
180
240
300
360
420
480
Zeit [min]


Abbildung 8

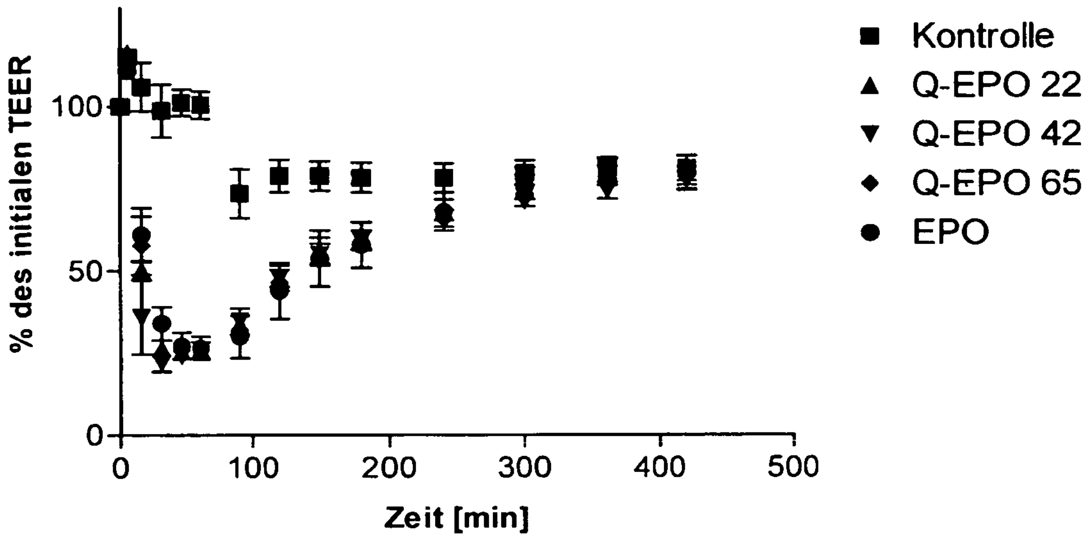

% des initialen TEER
100
50
0
0
100
200
300
400
500
Zeit [min]
Kontrolle
Q-EPO 22
Q-EPO 42
Q-EPO 65
EPO


Abbildung 9

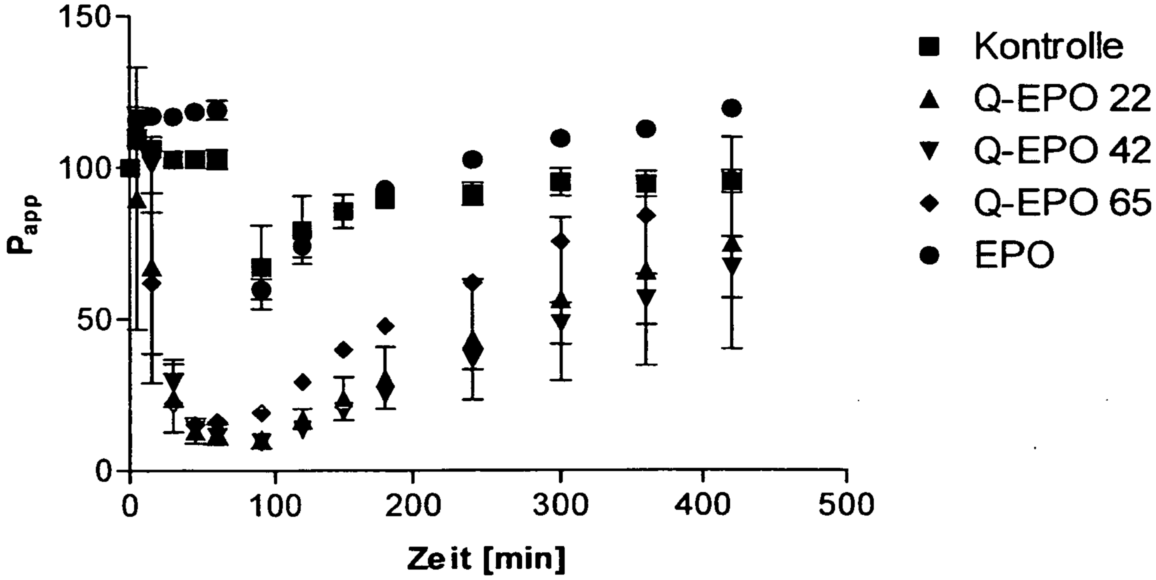


Abbildung 10

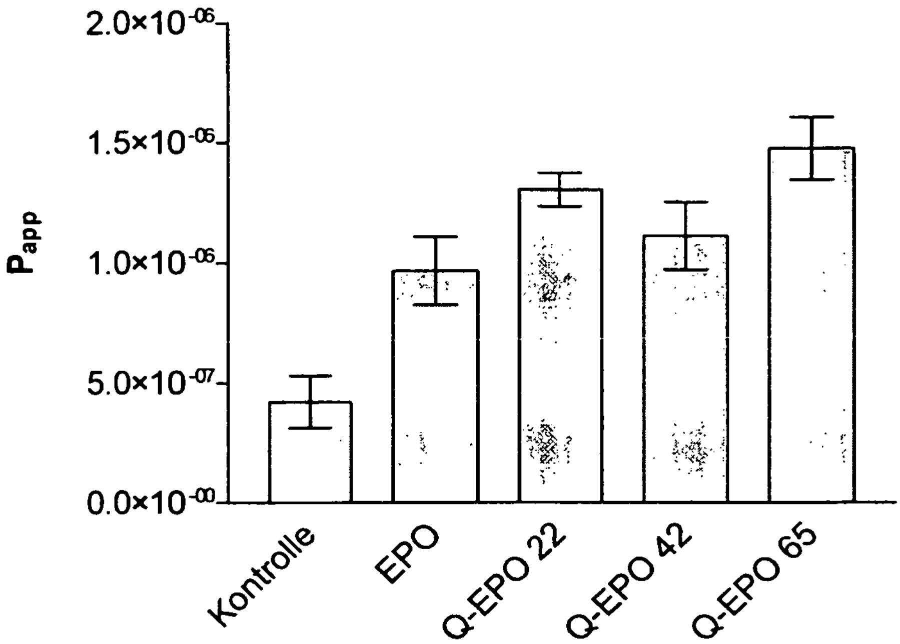


Abbildung 11

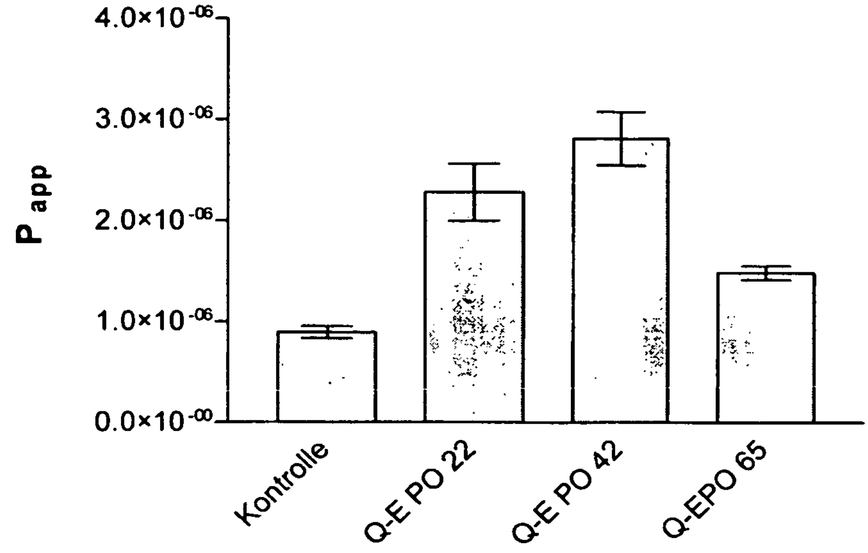


Abbildung 12

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1302201 A1


### In der Beschreibung aufgeführte Nicht-Patentliteratur


- *Current Drug Delivery,* 2005, vol. 2, 9-22
- *Macromol. Biosci.,* 2005, vol. 5, 207-213
- *J Pharm Sci.,* 2000, vol. 89, 63-75